(19) Europäisches Patentamt
European Patent Office
Office européen des brevets

(11) EP 0 969 791 B1

(12) EUROPÄISCHE PATENTSCHRIFT

(45) Veröffentlichungstag und Bekanntmachung des Hinweises auf die Patenterteilung:
**03.12.2003 Patentblatt 2003/49**

(21) Anmeldenummer: **98943728.0**

(22) Anmeldetag: **14.07.1998**

(51) Int Cl.[7]: **A61K 7/00**

(86) Internationale Anmeldenummer:
**PCT/EP98/04361**

(87) Internationale Veröffentlichungsnummer:
**WO 99/006013 (11.02.1999 Gazette 1999/06)**

(54) **MITTEL UND VERFAHREN ZUR DAUERHAFTEN HAARVERFORMUNG UND VERFAHREN ZUR HERSTELLUNG VON N-ALKYLMERCAPTOACETAMIDEN**

AGENT AND METHOD FOR PERMANENT SHAPING OF HAIR AND METHOD FOR THE PRODUCTION OF N-ALKYLMERCAPTOACETAMIDES

AGENT ET PROCEDE POUR LE PERMANENTAGE DES CHEVEUX ET PROCEDE POUR LA PRODUCTION DE L-ALKYLMERCAPTOACETAMIDES

(84) Benannte Vertragsstaaten:
**DE ES FR GB IT**

(30) Priorität: **31.07.1997 DE 29713619 U**

(43) Veröffentlichungstag der Anmeldung:
**12.01.2000 Patentblatt 2000/02**

(73) Patentinhaber: **Wella Aktiengesellschaft**
**64274 Darmstadt (DE)**

(72) Erfinder:
- **DANNECKER, Beate**
  **D-64283 Darmstadt (DE)**
- **LANG, Günther**
  **D-64354 Reinheim (DE)**
- **HANEFELD, Wolfgang**
  **D-35037 Marburg (DE)**
- **WALTHER, Heiko**
  **D-35037 Marburg (DE)**

(56) Entgegenhaltungen:

| | |
|---|---|
| **EP-A- 0 455 457** | **EP-A- 0 514 282** |
| **WO-A-91/10421** | **WO-A-95/31960** |
| **WO-A-98/30197** | **DE-A- 19 618 445** |
| **US-A- 4 220 602** | |

- **HAEFELE ET AL.: PR. SCIENT.SECT. TOILET GOODS ASSOC., Bd. 32, 1959, Seiten 52-57, XP002093017**

EP 0 969 791 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft ein Mittel zur dauerhaften Haarverformung auf der Basis von geradkettigen N-$C_3$- bis $C_6$-alkyl- oder N-$C_3$- bis $C_6$-hydroxyalkylsubstituierten Mercaptoacetamiden als keratinreduzierenden Wirkstoff sowie ein Verfahren zur dauerhaften Haarverformung unter Verwendung dieses Mittels.

Bekanntlich beruht die klassische Technik zur Durchführung einer dauerhaften Haarverformung auf zwei Behandlungsschritten: Im ersten Schritt werden die Cstin-Disulfid-Brücken des Haarkeratins durch Einwirken eines Mittels, welches einen reduzierenden Wirkstoff enthält (Verformungsmittel), geöffnet. Sodann wird das Haar in die gewünschte Form gebracht. In einem zweiten Schritt werden Cystin-Disulfid-Bindungen unter Verwendung eines Fixiermittels, d.h. eines einen oxidierenden Wirkstoff enthaltenden Mittels, wieder geschlossen.

Als klassisches Dauerwellreduktionsmittel wird, wie die Pionierarbeiten in den deutschen Patentschriften 948 186 und 972 424 zeigen, die Thioglykolsäure, z.B. als Ammonium- oder Monoethanolaminsalz, verwendet. Weitere übliche reduzierende Wirkstoffe sind anorganische Sulfite, 2-Mercapto-propionsäure (Thiomilchsäure), 3-Mercapto-propionsäure, Cystein und Derivate dieser Verbindungen sowie bestimmte Mercaptocarbonsäureester.

Alkalisch eingestellte Zubereitungen auf Basis der Mercaptocarbonsäuren zeigen trotz ausreichender Wirkung eine Haarschädigung, die sich beispielsweise in vermehrt auftretendem Haarbruch äußert. Vielfach belasten diese Mittel auch in unerwünschter Weise die Kopfhaut. Schließlich erfordert der unangehme Geruch der verwendeten Reduktionsmittel eine intensive Parfümierung der Produkte. Durch Verwendung von 2-Mercapto-propionsäure ist man in der Lage, einige der erwähnten Probleme zu lösen. Allerdings weist die Thiomilchsäure im Vergleich zur allgemein verwendeten Thioglykolsäure eine schwächere Umformung auf.

Für eine schonende, dauerhafte Umformung von geschädigtem, insbesondere gebleichtem oder gefärbten Haar werden bevorzugt schwach sauer bis neutral eingestellte Verformungsmittel verwendet. Hierbei haben sich aus fachlicher Sicht im Verlauf der letzten 35 Jahre die Thioglykolsäureester als für diesen Zweck am besten geeignete Reduktionsmittel erwiesen.

Ein erheblicher Nachteil von sauren Haarvertormungsmitteln auf der Basis von Thioglykolsäureestern ist jedoch deren schlechte Augen- und Hautverträglichkeit sowie die sensibiliserende Wirkung der Thioglykolsäureester, so daß auf einen Einsatz dieser Haarverformungsmittel heute weitgehend verzichtet wird. Anstelle der Mercaptocarbonsäureester wurden auch Mercaptocarbonsäureamide wie Thioglykolsäureamid oder alkyl- bzw. hydroxyalkylsubstituierte Amide verwendet. Derartige Verbindungen sind aus den Patentschriften DE-C-1 144 440 und EP-A-0 455 457 bekannt. Diese Stoffe haben, wie die Mercaptocarbonsäureester bei niedrigen pH-Werten ein hohes Umformungspotential, sind jedoch in Bezug auf die toxikologischen Eigenschaften noch kritischer als die Mercaptocarbonsäureester.

Der Erfindung liegt daher die Aufgabe zugrunde, ein Mittel und ein Verfahren zur dauerhaften Haarverformung zur Verfugung zu stellen, das sowohl im sauren als auch im schwach alkalischen Bereich (pH = 2 bis 9,5) , vor allem im pH-Bereich von 4,0 bis 8,5, eine schonende und gleichmäßige Umformung ermöglicht, kein oder ein nur ein geringes Sensibilsierungspotential besitzt und darüber hinaus ein stärkeres Umformungspotential als 2-Mercaptopropionsäure (Thiomilchsäure) aufweist.

Es wurde nun überraschend gefunden, daß sich die genannten Nachteile durch die Verwendung von bestimmten N-Alkylmercaptoacetamiden vermeiden lassen und daß dieses über ein stärkeres Umformungspotential als Thiomilchsäure verfügen.

Gegenstand der vorliegenden Erfindung ist daher ein Mittel zur dauerhaften Verformung der Haare, welches dadurch gekennzeichnet ist, daß es als keratinreduzierenden Wirkstoff ein N-Alkylmercaptoacetamid der Formel

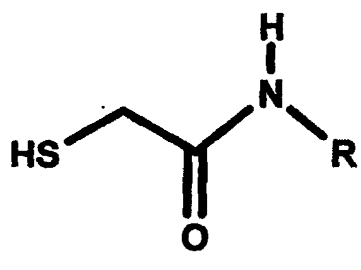
(I),

worin R einen geradkettigen Alkylrest mit 3 bis 6 Kohlenstoffatomen oder einen geradkettigen Hydroxyalkylrest mit 3 bis 6 Kohlenstoffatomen bedeutet, oder dessen Salz, enthält.

Bevorzugt ist R ein geradkettiger Alkylrest mit 3 Kohlenstoffatomen oder eine geradkettiger Hydroxyalkylrest

mit 3 Kohlenstoffatomen.

Vorzugsweise soll der geradkettige Hydroxyalkylrest eine oder zwei Hydroxylgruppen aufweisen.

Bevorzugte N-Alkylmercaptoacetamide der Formel (I) sind N-Propyl-2-mercaptoacetamid, N-(2'-Hydroxypropyl)-2-mercaptoacetamid und N-(3'-Hydroxypropyl)-2-mercaptoacetamid.

Als Salze kommen vor allem die Hydrogensalze mit den Säuren Salzsäure, Schwefelsäure, Essigsäure oder Zitronensäure in Betracht.

Das N-Alkylmercaptoacetamid der Formel (I) ist in dem Mittel bevorzugt als alleiniger keratinreduzierender Wirkstoff enthalten. Es kann jedoch auch in Kombination mit anderen keratinreduzierenden Wirkstoffen - wie zum Beispiel Thioglykolsäure, Thiomilchsäure, 2-Hydroxy-3-mercapto-propionsäure, Cysteamin und Cysteaminderivaten wie Alkyl- oder Acylcystamin oder Cystein und Cysteinderivaten, z. B. Cystein-(2-hydroxyethyl)-ester oder L-Cysteinglycerinester oder Sulfiten - eingesetzt werden.

Das N-Alkylmercaptoacetamid der Formel (I) wird in dem gebrauchsfertigen Mittel zur dauerhaften Haarverformung in einer Menge von 3 bis 28 Gewichtsprozent, vorzugsweise 5 bis 21 Gewichtsprozent, eingesetzt.

Die gebrauchsfertigen Haarverformungsmittel besitzen bevorzugt einen pH-Wert von 2,0 bis 9,5, besonders bevorzugt von 4,0 bis 8,5. Als Alkalisierungsmittel bzw. als Mittel zur Einstellung des pH-Wertes kommen insbesondere Ammoniak oder Natronlauge, aber auch wasserlösliche, physiologisch verträgliche Salze von organischen und anorganischen Basen, wie z.B. Ammoniumhydrogencarbonat, in Betracht. Zur Einstellung eines sauren pH-Wertes kann insbesondere Salzsäure, Essigsäure oder Zitronensäure verwendet werden.

Das Verformungsmittel kann sowohl ein- als auch zwei- oder dreikomponentig verpackt angeboten werden, wobei das Mittel in Form einer wäßrigen Lösung oder einer Emulsion als auch in verdickter Form auf wäßriger Basis, insbesondere als Creme, Gel, Schaum oder Paste vorliegen kann.

Selbstverständlich kann das Verformungsmittel alle für derartige Mittel üblichen und bekannten Zusatzstoffe, zum Beispiel Verdickungsmittel, wie beispielsweise Bentonit, Fettsäuren, Stärke, Polyacrylsäure und deren Derivate, Cellulosederivate, Alginate, Vaseline, Paraffinöle; Netzmittel oder Emulgatoren aus den Klassen der anionischen, kationischen, amphoteren oder nichtionogenen oberflächenaktiven Substanzen, beispielsweise Fettalkoholsulfate, Fettalkoholethersulfate, Alkylsulfonate, Alkylbenzolsulfate, quaternäre Ammoniumsalze, Alkylbetaine, oxethylierte Alkylphenole, Fettsäurealkanolamide oder oxethylierte Fettsäureester, ferner Trübungsmittel, wie zum Beispiel Polyethylenglykolester; Alkohole, wie zum Beispiel Ethanol, Propanol, Isopropanol, Polyole wie z. B. 1,2- oder 1,3-Propandiol, 1,2- , 1.3-oder 1,4-Butandiol, 1,2-Pentandiol und Glycerin; Zucker wie z. B. D-Glucose; Lösungsvermittler, Stabilisatoren, Puffersubstanzen, Parfümöle, Farbstoffe sowie haarkonditionierende und haarpflegende Bestandteile, wie zum Beispiel kationische Polymere, Lanolinderivate, Cholesterin, Pantothensäure und Betain, enthalten.

Die erwähnten Bestandteile werden in den für solche Zwecke üblichen Mengen verwendet, zum Beispiel die Netzmittel und Emulgatoren in Konzentrationen von insgesamt 0,2 bis 30 Gewichtsprozent, die Alkohole in einer Menge von insgesamt 0,1 bis 20 Gewichtsprozent, die Trübungsmittel, Parfümöle und Farbstoffe in einer Menge von jeweils 0,01 bis 1 Gewichtsprozent, die Puffersubstanzen in einer Menge von insgesamt 0,1 bis 10 Gewichtsprozent, Zucker, Lösungsvermittler, Stabilisatoren, sowie haarkonditionierende und haarpflegende Bestandteile in einer Menge von jeweils 0,1 bis 5 Gewichtsprozent, während die Verdickungsmittel und Lösungsvermittler in einer Menge von insgesamt 0,5 bis 20 Gewichtsprozent in diesem Mittel enthalten sein können.

Weiterhin können diesem Mittel zur Wirkungssteigerung sogenannte Quell- und Penetrationsstoffe, wie zum Beispiel Dipropylenglykolmonomethylether, 2-Pyrrolidon oder Imidazolidin-2-on, in einer Menge von 1 bis 30 Gewichtsprozent sowie zur Vermeidung einer Überkrausung der Haare Dithioverbindungen, beispielsweise Dithiodiglykoisäure, Dithiomilchsäure, die Disulfide der genannten Verbindungen oder deren Salze, zugesetzt werden.

Durch Variation des pH-Wertes kann ein Mittel zur Verfügung gestellt werden, das universell für jede Haarstruktur, gegebenenfalls unter zusätzlicher Anwendung von Wärme, geeignet ist. Das Mittel bewirkt eine elastische, dauerhafte und gleichmäßige Umformung vom Haaransatz bis zur Haarspitze, ohne allergische oder sensibilisierende Reaktionen hervorzurufen.

Die vorliegende Erfindung betrifft weiterhin ein Verfahren zur dauerhaften Haarverformung, bei dem man die Haare bevor und/oder nachdem man sie in die gewünschte Form bringt, mit einem Verformungsmittel behandelt, mit Wasser spült, sodann oxidativ behandelt, mit Wasser spült, gegebenenfalls zur Wasserwelle legt und sodann trocknet, welches dadurch gekennzeichnet ist, daß als Verformungsmittel die vorstehend beschriebenen erfindungsgemäßen Mittel verwendet werden.

Bei einer bevorzugten Ausführungsform des erfindungsgemäßen Verfahrens wird das Haar zunächst mit einem Shampoo gewaschen und danach mit Wasser gespült. Anschließend wird das handtuchtrockene Haar in einzelne Strähnen aufgeteilt und auf Wickler mit einem Durchmesser von 5 bis 30 Millimetern, bevorzugt 5 bis 15 Millimeter, gewickelt. Sodann wird das Haar mit einer für die Haarverformung ausreichenden Menge, vorzugsweise 60 bis 120 Gramm, des beschriebenen erfindungsgemäßen Verformungsmittels behandelt.

Nach einer für die dauerhafte Verformung des Haares ausreichenden Einwirkungszeit, welche je nach Haarbeschaffenheit, dem pH-Wert und der Verformungswirksamkeit des Verformungsmittels sowie in Abhängigkeit von der

Anwendungstemperatur 5 bis 30 Minuten (10 bis 30 Minuten ohne Wärmeeinwirkung; 5 bis 20 Minuten mit Wärmeeinwirkung) beträgt, wird das Haar mit Wasser gespült und dann oxidativ nachbehandelt ("fixiert"). Das Nachbehandlungsmittel wird, je nach Haarfülle, vorzugsweise in einer Menge von 80 bis 100 Gramm, verwendet.

Für die oxidative Nachbehandlung im aufgewickelten oder abgewickelten Zustand kann jedes beliebige, für eine derartige Behandlung geeignetes Nachbehandlungsmittel verwendet werden. Beispiele für in solchen Nachbehandlungsmitteln verwendbare Oxidationsmittel sind Kalium- und Natriumbromat, Natriumperborat, Harnstoffperoxid und Hydrogenperoxid. Die Konzentration des Oxidationsmittels ist in Abhängigkeit von der Anwendungszeit (in der Regel 5 bis 15 Minuten) und der Anwendungstemperatur unterschiedlich. Normalerweise liegt das Oxidationsmittel in dem gebrauchsfertigen wäßrigen Nachbehandlungsmittel in einer Konzentration von 0,5 bis 10 Gewichtsprozent vor. Das Mittel für die oxidative Nachbehandlung kann selbstverständlich weitere Stoffe, wie zum Beispiel Netzmittel, Pflegestoffe wie kationaktive Polymere, schwache Säuren, Puffersubstanzen oder Peroxidstabilisatoren, enthalten und in Form einer wäßrigen Lösung, einer Emulsion sowie in verdickter Form auf wäßriger Basis, insbesondere als Creme, Gel oder Paste, vorliegen. Diese üblichen Zusätze können insbesondere in einer Menge von 0,1 bis 10 Gew.% in dem Nachbehandlungsmittel enthalten sein.

Anschließend werden die Wickler entfernt. Falls erforderlich, kann das abgewickelte Haar nun nochmals oxidativ nachbehandelt werden. Sodann wird das Haar mit Wasser gespült, gegebenenfalls zur Wasserwelle gelegt und schließlich getrocknet.

Völlig überraschend weisen die bevorzugten N-Alkylmercaptoacetamide N-Propyl-2-mercaptoacetamid, N-(2'-Hydroxypropyl)-2-mercaptoacetamid und N-(3'-Hydroxypropyl)-2-mercaptoacetamid wesentlich geringere Sensibilisierungsraten auf, als die aus dem Stand der Technik nach DE-C-1 144 440 bzw. EP-A-0 455 457 als nächstliegende Dauerwellwirkstoffe bekannten N-Alkylmercaptoacetamide N-Methyl-mercaptoacetamid und N-Hydroxyethylmercaptoacetamid.

Die Herstellung des N-Alkylmercaptoacetamids der Formel (I) erfolgt durch Umsetzung des entsprechenden Amins bei einer Temperatur nicht über 30 Grad Celsius mit Methylthioglykolat unter Schutzgasatmosphäre, Extraktion in geeignetem Lösungsmittel und anschließende Kurzwegdestillation.

Die nachfolgenden Beispiele sollen den Gegenstand der Erfindung näher erläutern, ohne jedoch den Gegenstand auf diese Beispiele zu beschränken.

## HERSTELLUNGSBEISPIELE

### Beispiel 1 Herstellung von N-Propyl-2-mercaptoacetamid

In einem 500-ml-Dreihalskolben werden 118 g (2 mol) n-Propylamin vorgelegt. Es werden langsam 106,24 g Methylthioglykolat derart zugetropft, daß die Temperatur 30° C nicht übersteigt. Der Ansatz wird mit Argon durchspült und 2 Tage bei Raumtemperatur gerührt.

Das Gemisch wird unter Eiskühlung mit 36prozentiger Salzsäure angesäuert (pH 2 - 4) und mit Ethylacetat erschöpfend extrahiert. Das Lösungsmittel wird am Umlaufverdampfer im Vakuum abdestilliert, der Rückstand wird durch Zugabe von Natronlauge auf pH 7,0 gebracht und nochmals mit Ethylacetat ausgeschüttelt. Die vereinigten Fraktionen werden über Natriumsulfat getrocknet und eingeengt. Der vorliegende Rückstand wird mittels einer Kurzwegdestillationsapparatur bei höchstens 0,01 Torr zum reinen Produkt destilliert. Die Ausbeute beträgt 115 g (86 %).

| Analytik: | | |
|---|---|---|
| a) $^1$H-NMR ($CDCl_3$): | | |
| δ (ppm) = | 6,79 | (bauchig, -NH) |
| | 3,20 | (t, 2H, + NH-$CH_2$) |
| | 3,20 | (s, 2H, HS-$CH_2$-CO) |
| | 1,85 | (bauchig, 1H, HS) |
| | 1,52 | (m, 2H, NH-CH-$CH_2$) |
| | 0,90 | (t, 3H, $CH_2$-$CH_3$) |
| b) $^{13}$C-NMR ($CDCl_3$): | | |
| δ (ppm) = | 169,18 | (-C = O) |
| | 41,83 | (NH-CH) |
| | 28,36 | (HS-$CH_2$) |
| | 22,72 | (NH-CH-$CH_2$-$CH_3$) |

(fortgesetzt)

| Analytik: | | |
|---|---|---|
| b) $^{13}C$-NMR ($CDCl_3$): | | |
| | 11,37 | ($CH_2$-$CH_3$) |
| c) MS (70 e V, EI, RT) | | |
| | m/z (%) = | ($M^+$) = 133 (47,68)<br>100(29,1), 86 (24,58), 58 (8,6), 47 (25,67), 43 (100) |
| d) Thioltitration: 95,78 % | | |
| e) Elementaranalyse: $C_5H_{11}NOS$ | | (MG: 133,21) |
| | Ber.: C 45,08, H 8,32, N 10,51, S 24,07<br>Gef.: C 44,72, H 8,12, N 10,18, S 23,71 | |
| f) IR (NaCl-Platten): | | |
| | 3293s<br>3084-2876s<br>2552w<br>1652s<br>1559s | (NH)<br>($CH_2$)<br>(SH)<br>(N-monosubstituiertes Amid)<br>(N-monosubstituiertes Amid) |
| g) HPLC: | Die HPLC ergab ein Ergebnis von 98,07 Flächenprozent für die Verbindung | |
| | (Säule: C 18 5U, 250 mm x 4,6 mm; FließmittelAcetonitrit : Puffer [4 g $KH_2PO4$ + 0,8 g Octansulfonsäure-Na-Salz + 2 ml $H_3PO_4$] = 25 : 75 ; Flußrate 0,5 ml/min; Wellenlänge 200 nm;) | |
| h) pKs: | 8,451 ($H_2O$) | |
| i) UV-max: | 209,8 nm (Acetonitril: Puffer = 25 : 75) | |
| j) Siedepunkt: | 76 °C/0,01 Torr | |

**Beispiel 2 Herstellung von N-(2'-Hydroxypropyl)-2-mercaptoacetamid**

In einem 500-ml-Dreihalskolben werden 150,22 g (2 mol) 1-Amino-2-propanol vorgelegt. Es werden langsam 106,24 g (1 mol) Mercaptoessigsäuremethylester derart zugetropft, daß die Temperatur 30° C nicht übersteigt. Der Ansatz wird mit Argon durchspült und 2 Tage bei Raumtemperatur gerührt.

Das Gemisch wird unter Eiskühlung mit 36prozentiger Salzsäure angesäuert (pH 2 - 4) und mit Ethylacetat erschöpfend extrahiert. Das Lösungsmittel wird am Umlaufverdampfer im Vakuum abdestilliert, der Rückstand wird durch Zugabe von Natronlauge auf pH 7,0 gebracht und nochmals mit Ethylacetat ausgeschüttet. Die vereinigten Fraktionen werden über Natriumsulfat getrocknet und eingeengt. Der vorliegende Rückstand wird mittels einer Kurzwegdestillationsapparatur bei höchstens 0,01 Torr zum reinen Produkt destilliert. Die Ausbeute beträgt 95 g (64 %).

| Analytik: | | | |
|---|---|---|---|
| a) $^1H$-NMR ($CDCl_3$): | | | |
| $\delta_H$ (ppm) = | 6,70<br>3,93<br>3,44 und 3,14<br>3,26<br>2,07<br>1,21 | (bauchig, -NH)<br>(m, 1H, CH)<br>(m, jeweils 1 H, NH-$\underline{CH_2}$)<br>(s, 2H, HS-$\underline{CH_2}$-CO)<br>(t, 1 H, HS)<br>(d, 3H, $CH_3$) | |
| b) $^{13}C$-NMR ($CDCl_3$): | | | |
| $\delta_c$ (ppm) = | 170,71<br>67.00 | (-C=O)<br>(NH-$CH_2$-$\underline{CH}$) | |

(fortgesetzt)

| Analytik: | | | |
|---|---|---|---|
| b) $^{13}$C-NMR ($CDCl_3$): | | | |
| | 47,38<br>28,24<br>20,87 | (NH-$\underline{CH_2}$)<br>(HS-$\underline{CH_2}$)<br>($\underline{CH_3}$) | |
| c) MS (70 e V, EI, RT) | | | |
| m/z (%) = | ($M^+$) = 149 (8,39)<br>131(47,08), 105 (100), 84 (27,49), 72 (72,76), 58 (57,75), 56 (32,11), 47 (59,94) | | |
| d) Thioltitration: 97,88 % | | | |
| e) Elementaranalyse: $C_6H_{13}NOS$ | | | (MG: 149,21) |
| | Ber.: C 40,25, H 7,43, N 9,39, S 21,49<br>Gef.: C 40,11, H 7,42, N 9,12, S 21,53 | | |
| f) IR (KBr): | | | |
| | 3314s<br>3090-2927s<br>2549w<br>1657s<br>1554s | (OH)<br>($CH_2$)<br>(SH)<br>(N-monosubstituiertes Amid)<br>(N-monosubstituiertes Amid) | |
| g) HPLC: | Die HPLC ergab ein Ergebnis von 98,88 Flächenprozent für die Verbindung. | | |
| | (Säule: C 18 5U, 250 mm x 4,6 mm; FließmittelAcetonitril : Puffer [4 g $KH_2PO4$ + 0,8 g Octansulfonsäure-Na-Salz + 2 ml $H_3PO_4$] = 25 : 75 ; Flußrate 0,5 ml/min; Wellenlänge 200 nm;) | | |
| h) pKs: | 7,865 ($H_2O$) | | |
| i) UV-max: | 218,0 nm (Acetonitril: Puffer = 25 : 75) | | |
| j) Siedepunkt: | 108 °C/0,01 Torr | | |

**Beispiel 3 Herstellung von N-(3'-Hydroxypropyl)-2-mercaptoacetamid**

In einem 500-ml-Dreihalskolben werden 150,22 g (2 mol) 3-Amino-1-propanol vorgelegt. Es werden langsam 106,24 g (1 mol) Mercaptoessigsäuremethylester derart zugetropft, daß die Temperatur 30° C nicht übersteigt. Der Ansatz wird mit Argon durchspült und 2 Tage bei Raumtemperatur gerührt.

Das Gemisch wird unter Eiskühlung mit 36prozentiger Salzsäure angesäuert (pH 2 - 4) und mit Ethylacetat erschöpfend extrahiert. Das Lösungsmittel wird am Umlaufverdampfer im Vakuum abdestilliert, der Rückstand wird durch Zugabe von Natronlauge auf pH 7,0 gebracht und nochmals mit Ethylacetat ausgeschüttelt. Die vereinigten Fraktionen werden über Natriumsulfat getrocknet und eingeengt. Der vorliegende Rückstand wird mittels einer Kurzwegdestillationsapparatur bei höchstens 0,01 Torr zum reinen Produkt destilliert. Die Ausbeute beträgt 63 g (42 %).

| Analytik: | | | |
|---|---|---|---|
| a) $^1$H-NMR ($CDCl_3$): | | | |
| $\delta_H$ (ppm) = | 7,062<br>3,64<br>3,42<br>3,22<br>2,04 | (bauchig, -NH)<br>(m, 2H, $CH_2$-OH)<br>(m, 2H, $\underline{CH_2}$-$CH_2$-$CH_2$-OH)<br>(d, 2H, HS-$\underline{CH_2}$-CO)<br>(t, 1H, $CH_2$-$\underline{OH}$) | |

(fortgesetzt)

| | | | |
|---|---|---|---|
| Analytik: | | | |
| a) $^{1}H$-NMR ($CDCl_3$): | | | |
| | 1,72<br>1,69 | (t, 1H, HS)<br>(m, 2H, $CH_2$-$\underline{CH_2}$-$CH_2$-OH) | |
| b) $^{13}C$-NMR ($CDCl_3$): | | | |
| $\delta_c$ (ppm) = | 170,59<br>59,86<br>37,06<br>31,99<br>28,53 | (-C=O)<br>(NH-$CH_2$)<br>($\underline{CH2}$-OH)<br>($CH_2$-$\underline{CH_2}$-$CH_2$-OH)<br>(HS-$\underline{CH_2}$) | |
| c) MS (70 e V, EI, RT) | | | |
| m/z (%) = | ($M^+$) = 149 (36,13)<br>131(36,58), 116 (31,62), 105 (23,15), 102 (100), 84 (72,8), 76 (24,49), 56 (66,29) | | |
| d) Thioltitration: | 94,063 % | | |
| e) Elementaranalyse: $C_5H_{11}NOS$ | | | (MG: 149,21) |
| Ber.: | C 40,25, H 7,43, N 9,39, S 21,49 | | |
| Gef.: | C 40,21, H 6,99, N 9,05, S 21,53 | | |
| f) IR (NaCl): | | | |
| | | 3299s<br>3086-2918s<br>2546w<br>1651 s<br>1551s | (NH)<br>($CH_2$)<br>(SH)<br>(N-monosubstituiertes Amid)<br>(N-monosubstituiertes Amid) |
| g) HPLC: | Die HPLC ergab ein Ergebnis von 93,19 Flächenprozent für die Verbindung. | | |
| | (Säule: C 18 5U, 250 mm x 4,6 mm; FließmittelAcetonitril : Puffer [4 g $KH_2PO4$ + 0,8 g Octansulfonsäure-Na-Salz + 2 ml $H_3PO_4$] = 25 : 75 ; Flußrate 0,5 ml/min; Wellenlänge 200 nm;) | | |
| h) pKs: | 8,406 ($H_2O$) | | |
| i) UV-max: | 223,2 nm (Acetonitril: Puffer = 25 : 75) | | |
| j) Siedepunkt: | 115 °C/0,01 Torr | | |

**Beispiel 4 Vergleich der Wellwirksamkeit**

Die Wellwirksamkeit der Verbindung wurde unter Verwendung von Glycerinmonothioglykolat als Vergleichssubstanz mit Hilfe von Wellösungen bei pH = 7, 8 und 9 bestimmt. Hierzu wurden 16,5 Zentimeter lange, vorgebleichte und damit geschädigte Zähthaarsträhnen (bestehend aus ca. 100 Haaren) aus mitteleuropäischem Haar naß auf genormte Spiralwickler (Innendurchmesser: 3 Millimeter) aufgewickelt und nach dem Konditionieren im Klimaraum (Temperatur: 20°C; Luftfeuchte: 65%) mit einer 87 mmol/100g enthaltenden, auf den jeweiligen pH-Wert eingestellten Lösung der Reduktionsmittel behandelt. Die Auftragemenge an Wellflüssigkeit wurde über das Verhältnis 1 : 1,2 errechnet (1g Haar : 1,2 ml Wellflüssigkeit). Als Einwirkzeit wurden 20 Minuten gewählt; die Einwirktemperatur betrug 50 Grad Celsius. Anschließend wurden die Haare mit einer peroxidhaltigen Fixierung fixiert, getrocknet und nach dem Abwickeln vier Stunden in Wasser (Wasserbadtemepratur: 40°C) ausgehängt.

Die Wellstabilität errechnet sich gemäß folgender Formel:

$$\text{Wellstabilität in \%} = \frac{l_o - l_t}{l_o - l_1} \times 100$$

$l_o$ = Gesamtlänge der nicht umgeformten, gestreckten Strähne (16,5 Zentimeter)
$l_t$ = Länge der abgewickelten, ausgehängten Strähne nach 240 Minuten
$l_1$ = Länge der umgeformten, aufgewickelten Strähne (bei einem Wickelinnendurchmesser von 3 Millimeter beträgt diese 35 Millimeter)

Tabelle 1

| Wellwirkstoff | Ausbeute in % | Elementaranalyse berechnet/ gefunden | HPLC (FP) | Sp. | WSH pH=7 in % | WSN pH = 8 in % | WSN pH = 9 in % |
|---|---|---|---|---|---|---|---|
| N-Propyl-2-mercapto-acetamid | 86 | C: 45,08, H: 8,32, N: 10,51, S: 24,07, C: 44,72, H: 8,12, N: 10,18, S: 23,71 | 98,069 | 76° C 0,01 Torr | 85 | 96 | 100 |
| N-(2'-Hydroxy-propyl)-2-mercaptoacetamid | 64% | C: 40,25, H: 7,43, N: 9,39, S: 21,49, C: 40,25, H: 7,30, N: 9,16, S: 20,63 | 98,88 | 108° C 0,01 Torr | 92 | 97 | 97 |
| N-(3'-Hydroxy-propyl)-2-mercapto-acetamid | 42% | C: 40,25, H: 7,43, N: 9,39, S: 21,49, C: 40,21, H: 6,99, N: 9,05, S: 21,32 | 93,19 | 115° C 0,01 Torr | 84 | 89 | 99 |
| Thiomilchsäure (2-Mercaptopropionsäure) als Vergleich | | | | | 38 | 54 | 62 |

Als Standard wurden Strähnchen mit einer entsprechend auf pH 9 eingestellten Glycerinmonothioglykolat-Lösung behandelt. Die vorstehend in Tabelle 1 angegebenen normierten Wellstabilitäten (WSN) beziehen sich auf diese Standardiösung (pH = 9), deren Wellstabilität auf 100 Prozent gesetzt wurde.

Tabelle 1 zeigt, daß die Wellwirksamkeiten der erfindungsgemäßen Mercaptoacetamide bei pH 7, 8 und 9 höher sind als bei Thiomilchsäure.

BEISPIELE FÜR DAUERVERFORMUNGSMITTEL

**Beispiel 5 Dauerverformungsmittel für gefärbtes Haar**

| | |
|---|---|
| 10,8 g | N-Propyl-2-mercaptoacetamid |
| 0,4 g | Ammoniak (25%-ige wäßrige Lösung) zur pH-Einstellung |
| 2,0 g | Ammoniumhydrogencarbonat |
| 5,0 g | Isopropanol |
| 2,5 g | 1,2-Propylenglykol |
| 2,5 g | 1,2-Pentandiol |
| 1,0 g | Isooctylphenol, oxethyliert mit 10 Mol Ethylenoxid |
| 1,0 g | Poly(dimethyldiallylammoniumchlorid) |
| 0,3 g | Parfümöl |

(fortgesetzt)

| | |
|---|---|
| 0,1 g | Vinylpyrrolidon/Styrol-Mischpolymerisat (Antara® 430 der GAF Corp.; New York/USA) |
| 74,4 g | Wasser |
| 100,0 g | |

Der pH-Wert dieses Mittels liegt bei 7,3.

Durch Farbbehandlungen vorgeschädigtes Haar wird mit einem Shampoo gewaschen, frottiert und auf Wickler mit einem Durchmesser von 8 Millimetern gewickelt. Anschließend wird das vorstehend beschriebene Haarverformungsmittel gleichmäßig auf dem gewickelten Haar verteilt. Sodann wird das Haar mit einer Plastikhaube abgedeckt und 10 Minuten lang unter einer Trockenhaube bei einer Temperatur von 45 Grad Celsius erwärmt. Anschließend wird die Abdeckung entfernt, das Haar mit Wasser gespült und mit 100 Gramm einer 3-prozentigen wäßrigen Hydrogenperoxidlösung oxidativ nachbehandelt. Nach Entfernung der Wickler werden die Haare erneut mit Wasser gespült, zur Wasserwelle gelegt und sodann getrocknet.

Als Ergebnis dieser Behandlung wird eine gleichmäßige, elastische und dauerhafte Verformung der Haare erhalten.

**Beispiel 6 Dauerwellmittel für normales Haar**

| | |
|---|---|
| 14,5 g | N-Propyl-2-mercaptoacetamid |
| 8,9 g | Ammoniak (25%-ige wäßrige Lösung) |
| 4,0 g | Ammoniumhydrogencarbonat |
| 1,0 g | 1,3-Butandiol |
| 5,0 g | 1,2-Propylenglykol |
| 2,0 g | Dipropylenglykolmonoethylether |
| 4,0 g | Harnstoff |
| 2,5 g | hydriertes Rizinusöl, oxethyliert mit 40 Mol Ethylenoxid |
| 2,5 g | Laurylakohol, oxethyliert mit 4 Mol Ethylenoxid (CTFA: Laureth-4) |
| 0,5 g | Parfümöl |
| 0,1 g | Vinylpyrrolidon/Styrol-Mischpolymerisat (Antara® 430 der GAF Corp.; New York/USA) |
| 55,0 g | Wasser |
| 100,0 g | |

Der pH-Wert dieses Mittels beträgt 8,4.

Normales, nicht vorgeschädigtes Haar wird gewaschen, mit einem Handtuch frottiert und auf Wickler mit einem Durchmesser von 6 Millimetern gewickelt. Anschließend wird das Haar mit dem vorstehend beschriebenen Haarverformungsmittel gleichmäßig durchfeuchtet. Nach einer Einwirkungszeit von 15 Minuten wird das Haar mit Wasser gründlich gespült und sodann mit 80 Gramm einer 3-prozentigen wäßrigen Hydrogenperoxidlösung oxidativ nachbehandelt. Nach Entfernung der Wickler werden die Haare erneut mit Wasser gespült, zur Wasserwelle gelegt und anschließend getrocknet. Das so behandelte Haar besitzt eine gleichmäßige und lebhafte Krause.

**Beispiel 7 Dauerverformungsmittel für normales Haar**

| | |
|---|---|
| 7,7 g | N-Propyl-2-mercaptoacetamid |
| 8,7 g | N-(2'-Hydroxypropyl)-2-mercaptoacetamid |
| 8,9 g | Ammoniak (25%-ige wäßrige Lösung) zur pH-Einstellung |
| 5,0 g | Ammoniumhydrogencarbonat |
| 1,0 g | 1,2-Propandiol |

(fortgesetzt)

| | |
|---|---|
| 2,0 g | D-Glucose |
| 2,4 g | Ammoniak |
| 1,5 g | Isooctylphenol, oxethyliert mit 10 Mol Ethylenoxid |
| 0,5 g | Poly(dimethyldiallylammoniumchlorid) |
| 0,5 g | Parfümöl |
| 0,1 g | Vinylpyrrolidon/Styrol-Mischpolymerisat |
| | (Antara® 430 der GAF Corp.; New York/USA) |
| 61,7 g | Wasser |
| 100,0 g | |

Der pH dieses Mittels liegt bei 8,3.

Normales, nicht vorgeschädigtes Haar wird gewaschen, mit einem Handtuch frottiert und auf Wickler mit einem Durchmesser von 6 Millimetern gewickelt. Anschließend wird das Haar mit dem vorstehend beschriebenen Haarverformungsmittel gleichmäßig durchfeuchtet. Nach einer Einwirkungszeit von 15 - 25 Minuten wird das Haar mit Wasser gründlich gespült und sodann mit 80 Gramm einer 3-prozentigen wäßrigen Hydrogenperoxidlösung oxidativ nachbehandelt. Nach Entfernung der Wickler werden die Haare erneut mit Wasser gespült, zur Wasserwelle gelegt und anschließend getrocknet. Das so behandelte Haar besitzt eine gleichmäßige und lebhafte Krause.

**Beispiel 8 zweikomponentiges Dauerverformungsmittel für normales Haar**

Komponente A

| | |
|---|---|
| 4,5 g | Ammoniumhydrogencarbonat |
| 0,4 g | Ammoniak, 25%ig zur pH-Einstellung |
| 2,0 g | Diethylenglykolmonoethylether |
| 2,0 g | 1-Methoxypropanol |
| 1,5 g | Propylenglykol |
| 0,5 g | Oleylalkoholpolyethylenglykolether-5EO (CTFA: OLETH-5) |
| 0,5 g | quaternäres Ammoniumsalz des Terpolymers aus Acrylsäure/ Diallyldimethylammoniumchlorid/ Acrylamid (CTFA: POLYQUATERNIUM-39) Handelsprodukt: Merquat® Plus 3330 |
| 0,5 g | Parfümöl |
| 88,1 g | Wasser |
| 100,0 g | |

Komponente B

| | |
|---|---|
| 50,0 g | N-(2'-Hydroxypropyl)-2-mercaptoacetamid, 94%ig |

Für die Anwendung des zweikomponentigen Dauerwellmittels werden 60 g der Komponente A mit 14 g der Komponente B zu einem gebrauchsfertigen Haarverformungsmittel vermischt. Beim Mischen entsteht ein Produkt mit dem pH-Wert 6,5.

Normales, nicht vorgeschädigtes Haar wird gewaschen, mit einem Handtuch frottiert und auf Wickler mit einem Durchmesser von 6 Millimetern gewickelt. Anschließend wird das Haar mit dem vorstehend beschriebenen gebrauchsfertigen Haarverformungsmittel gleichmäßig durchfeuchtet. Nach einer Einwirkungszeit von 15 - 25 Minuten wird das Haar mit Wasser gründlich gespült und sodann mit 80 Gramm einer 3-prozentigen wäßrigen Hydrogenperoxidlösung oxidativ nachbehandelt. Nach Entfernung der Wickler werden die Haare erneut mit Wasser gespült, zur Wasserwelle gelegt und anschließend getrocknet. Das so behandelte Haar besitzt eine gleichmäßige und lebhafte Krause und liefert eine exzellent ausgebildete, stabile Dauerwellung.

**Beispiel 9 Dauerverformungsmittel für normales Haar**

| | |
|---|---|
| 7,7 g | N-Propyl-2-mercaptoacetamid |
| 8,7 g | N-(3'-Hydroxypropyl)-2-mercaptoacetamid |
| 5,0 g | Isopropylalkohol |
| 1,0 g | 1,2-Propandiol |
| 0,5 g | Kokosfettalkohol, mit 10 Mol Ethylenoxid oxethyliert |
| 0,5 g | Parfümöl |
| 0,1 g | Vinylpyrrolidon/Styrol-Mischpolymerisat (Antara® 430 der GAF Corp.; New York/USA) |
| 76,5 g | Wasser, vollentsalzt |
| 100,0 g | |

Der pH dieses Mittels liegt bei 4,5.

Normales, nicht vorgeschädigtes Haar wird gewaschen, mit einem Handtuch frottiert und auf Wickler mit einem Durchmesser von 6 Millimetern gewickelt. Anschließend wird das Haar mit dem vorstehend beschriebenen Haarverformungsmittel gleichmäßig durchfeuchtet. Nach einer Einwirkungszeit von 15 - 25 Minuten wird das Haar mit Wasser gründlich gespült und sodann mit 80 Gramm einer 3-prozentigen wäßrigen Hydrogenperoxidlösung oxidativ nachbehandelt. Nach Entfernung der Wickler werden die Haare erneut mit Wasser gespült, zur Wasserwelle gelegt und anschließend getrocknet. Das so behandelte Haar besitzt eine über die gesamte Haarlänge gleichmäßige Krause, die mit der durch Behandlung mit mildalkalischen Dauerverformungsmitteln erzielten Krause vergleichbar ist.

**Belspiel 10 Dauerverformungsmittel für gefärbtes Haar**

| | |
|---|---|
| 13,7 g | N-(3'-Hydroxypropyl)-2-mercaptoacetamid, 90%ig |
| 4,4 g | Ammoniak (25%-ige wäßrige Lösung) zur pH-Einstellung |
| 2,0 g | Ammoniumhydrogencarbonat |
| 2,0 g | Dipropylenglykolmonoethylester |
| 2,5 g | 1,2-Propylenglykol |
| 1,0 g | Poly(dimethyldiallylammoniumchlorid) |
| 0,3 g | Parfümöl |
| 2,5 g | Phosphorsäureester des mit 4 Mol Ethylenoxid oxethylierten Decylalkohols (CTFA: DECETH-4 PHOSPHATE) |
| 71,6 g | Wasser |
| 100,0 g | |

Der pH-Wert dieses Mittels liegt bei 7,3.

Durch Farbbehandlungen vorgeschädigtes Haar wird mit einem Shampoo gewaschen, frottiert und auf Wickler mit einem Durchmesser von 8 Millimetern gewickelt. Anschließend wird das vorstehend beschriebene Haarverformungsmittel gleichmäßig auf dem gewickelten Haar verteilt. Sodann wird das Haar mit einer Plastikhaube abgedeckt und 10 Minuten lang unter einer Trockenhaube bei einer Temperatur von 45 Grad Celsius erwärmt. Anschließend wird die Abdeckung entfernt, das Haar mit Wasser gespült und mit 100 Gramm einer 3-prozentigen wäßrigen Hydrogenperoxidlösung oxidativ nachbehandelt. Nach Entfernung der Wickler werden die Haare erneut mit Wasser gespült, zur Wasserwelle gelegt und sodann getrocknet.

Als Ergebnis dieser Behandlung wird eine gleichmäßige, elastische und dauerhafte Verformung der Haare erhalten.

## Patentansprüche

1. Mittel zur dauerhaften Verformung von Haaren, **dadurch gekennzeichnet, daß** es als keratinreduzierenden Wirkstoff ein N-Alkylmercaptoacetamid der Formel

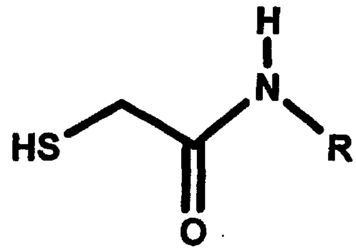

(I),

worin R einen geradkettigen Alkylrest mit 3 bis 6 Kohlenstoffatomen oder einen geradkettigen Hydroxyalkylrest mit 3 bis 6 Kohlenstoffatomen bedeutet, oder dessen Salz, enthält.

2. Mittel nach Anspruch 1, **dadurch gekennzeichnet, daß** das N-Alkylmercaptoacetamid der Formel (I) ausgewählt ist unter N-Propyl-2-mercaptoacetamid, N-(2'-Hydroxypropyl)-2-mercaptoacetamid und N-(3'-Hydroxypropyl)-2-mercaptoacetamid.

3. Mittel nach einem der Ansprüche 1 oder 2, **dadurch gekennzeichnet, daß** das N-Alkylmercaptoacetamid der Formel (I) in dem gebrauchsfertigen Mittel in einer Menge von 3 bis 28 Gewichtsprozent enthalten ist.

4. Mittel nach einem der Ansprüche 1 bis 3, **dadurch gekennzeichnet, daß** der pH-Wert des gebrauchsfertigen Mittels 4 bis 8,5 beträgt.

5. Mittel nach einem der Ansprüche 1 bis 4, **dadurch gekennzeichnet, daß** es vor der Anwendung durch Vermischen von zwei oder drei Komponenten erhalten wird.

6. Verfahren zur dauerhaften Verformung von Haaren, bei dem man das Haar bevor und/oder nachdem man es in der gewünschten Form festhält, mit einem Verformungsmittel behandelt, mit Wasser spült, oxidativ nachbehandelt, erneut mit Wasser spült, gegebenenfalls zur Wasserwelle legt und sodann trocknet, **dadurch gekennzeichnet, daß** man als Verformungsmittel ein Mittel nach einem der Ansprüche 1 bis 4 verwendet.

7. Verfahren nach Anspruch 6, **dadurch gekennzeichnet, daß** man das Verformungsmittel 5 bis 30 Minuten lang einwirken läßt.

8. Verfahren nach Anspruch 6 oder 7, **dadurch gekennzeichnet, daß** man das Verformungsmittel unter Anwendung von Wärme 5 bis 20 Minuten lang einwirken läßt.

9. Verfahren nach einem der Ansprüche 6 bis 9, **dadurch gekennzeichnet, daß** man das Verformungsmittel in einer Menge von 60 bis 120 Gramm anwendet.

## Claims

1. Agent for the permanent shaping of hair, **characterized in that** it comprises, as keratin-reducing active ingredient, an N-alkylmercaptoacetamide of the formula

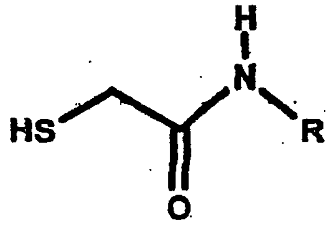

(I),

in which R is a straight-chain alkyl radical having 3 to 6 carbon atoms or a straight-chain hydroxyalkyl radical having 3 to 6 carbon atoms, or salt thereof.

2. Agent according to Claim 1, **characterized in that** the N-alkylmercaptoacetamide of the formula (I) is chosen from N-propyl-2-mercaptoacetamide, N-(2'-hydroxypropyl)-2-mercaptoacetamide and N-(3'-hydroxypropyl)-2-mercaptoacetamide.

3. Agent according to one of Claims 1 or 2, **characterized in that** the N-alkylmercaptoacetamide of the formula (I) is present in the ready-to-use agent in an amount of from 3 to 28 per cent by weight.

4. Agent according to one of Claims 1 to 3, **characterized in that** the pH of the ready-to-use agent is 4 to 8.5.

5. Agent according to one of Claims 1 to 4, **characterized in that** it is obtained prior to use by mixing two or three components.

6. Method for the permanent shaping of hair, in which the hair, before and/or after it is held in the desired shape, is treated with a shaping agent, rinsed with water, oxidatively after-treated, rinsed again with water, optionally arranged in a water-wave and then dried, **characterized in that** the shaping agent used is an agent according to one of Claims 1 to 4.

7. Method according to Claim 6, **characterized in that** the shaping agent is left to act for 5 to 30 minutes.

8. Method according to Claim 6 or 7, **characterized in that** the shaping agent is left to act for 5 to 20 minutes with the application of heat.

9. Method according to one of Claims 6 to 9, **characterized in that** the shaping agent is applied in an amount of from 60 to 120 grams.

## Revendications

1. Composition pour la mise en forme permanente des cheveux, **caractérisée en ce qu'**elle contient en tant que substance active réduisant la kératine un N-alkylmercaptoacétamide de formule

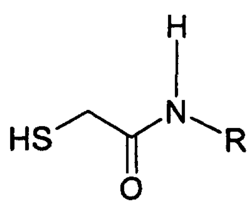

(I),

dans laquelle R représente un radical alkyle à chaîne droite ayant de 3 à 6 atomes de carbone ou un radical hydroxyalkyle à chaîne droite ayant de 3 à 6 atomes de carbone, ou un sel d'un tel composé.

2. Composition selon la revendication 1, **caractérisée en ce que** le N-alkylmercaptoacétamide de formule (I) est choisi parmi le N-propyl-2-mercaptoacétamide, le N-(2'-hydroxypropyl)-2-mercaptoacétamide et le N-(3'-hydroxypropyl)-2-mercaptoacétamide.

3. Composition selon la revendication 1 ou 2, **caractérisée en ce que** le N-alkylmercaptoacétamide de formule (I) est contenu dans la composition prête à l'emploi en une quantité de 3 à 28 % en poids.

4. Composition selon l'une quelconque des revendications 1 à 3, **caractérisée en ce que** le pH de la composition prête à l'emploi est de 4 à 8,5.

5. Composition selon l'une quelconque des revendications 1 à 4, **caractérisée en ce qu'**elle est obtenue avant l'emploi, par mélange de deux ou trois composants.

6. Procédé pour la mise en forme permanente des cheveux, dans lequel les cheveux, avant et/ou après avoir été maintenus sous la forme désirée, sont traités par une composition de mise en forme, rincés à l'eau, soumis à un après-traitement par oxydation, rincés à nouveau à l'eau, éventuellement mis en plis et ensuite séchés, **caractérisé en ce qu'**on utilise comme composition de mise en forme une composition selon l'une quelconque des revendications 1 à 4.

7. Procédé selon la revendication 6, **caractérisé en ce qu'**on laisse agir la composition de mise en forme pendant 5 à 30 minutes.

8. Procédé selon la revendication 6 ou 7, **caractérisé en ce qu'**on laisse agir la composition de mise en forme, avec application de chaleur, pendant 5 à 20 minutes.

9. Procédé selon l'une quelconque des revendications 6 à 9, **caractérisé en ce que** la composition de mise en forme est utilisée en une quantité de 60 à 120 grammes.